# EUROPEAN PATENT APPLICATION

(11) **EP 4 520 380 A1**
(43) Date of publication of application: **12.03.2025**
(21) Application number: 23799095.7
(22) Date of filing: 10.02.2023
(51) Int. Cl.: A61M 25/10

(54) **BALLOON CATHETER APPARATUS AND SYSTEM FOR EXPANDING AORTIC VALVE**

(30) Priority: 05.05.2022 CN 202210483724
(71) Applicant: Shanghai Trulive Medtech Co., Ltd., Shanghai 201206 (CN); Jiangsu Trulive Medtech Co., Ltd., Nantong, Jiangsu 226400 (CN)
(72) Inventor: JIANG, Fei, Shanghai 201206 (CN); SUN, Yunyan, Shanghai 201206 (CN); WEI, Yongqiang, Shanghai 201206 (CN)
(74) Representative: Patentship Patentanwaltsgesellschaft mbH
(86) International application number: PCT/CN2023/075317
(87) International publication number: WO 2023/213105

(57) **Abstract**

The present invention provides a balloon catheter apparatus for dilating an aortic valve, which includes a catheter assembly and an expandable assembly. The expandable assembly includes three independent expandable members arranged in a direction from a distal end to a proximal end of the catheter assembly. A middle expandable member is configured for placement of a prosthetic valve thereon so that the prosthetic valve surrounds the the middle expandable member. The catheter assembly includes a distal catheter, a middle catheter and a proximal catheter, each of which is configured to transfer an inflation medium. The distal catheter communicates with an inflation cavity of the distal expandable member, the middle catheter communicates with the middle expandable member, and the proximal catheter communicates with a proximal one of the expandable members. According to the present invention, the expandable members can be independently controlled to expand in a desired order to desired degrees to allow for more precise deployment control of a prosthetic valve in terms of both location and shape. This provides for deployment shape modification and optimization by a surgeon according to a patient's anatomical characteristics.

## Description

### TECHNICAL FIELD

The present invention relates to the field of medical technology, and particularly to a balloon catheter apparatus for dilating an aortic valve and a system.

### BACKGROUND

Transcatheter aortic valve replacement (TAVR) involves threading an interventional catheter through the femoral artery and advancing a prosthetic heart valve to a target aortic valve. Unfolding the prosthetic heart valve in order to complete the implantation procedure of the prosthesis valve, and to recover the function of the valve. As this procedure does not involve thoracotomy, it is less traumatic and allows fast postoperative recovery.

Conventionally, TAVR often relies on balloon-assisted expansion or self-expansion for the unfolding and deployment of a prosthetic valve. In the former case, a prosthetic valve may be crimped on an expandable balloon and delivered in a bendable sheath tube to the vicinity of the native aortic valve, and the balloon may be then inflated with water, air or the like to unfold the crimped prosthetic valve.

Existing expandable balloons are usually integral single-piece balloons dividable into three parts: distal, proximal and waist. The waist part is generally straight or recessed. Supports are provided internally between the distal and proximal parts of a balloon, and the prosthetic valve can be crimped on the waist part and located between the supports. The supports are used to restrict the crimped prosthetic valve from unwanted axial displacement when it is being advanced for delivery, which may make it unsuccessful to dilate the valve by expanding the balloon because of the displacement of the valve.

As these balloons are integral structures, the distal, proximal and waist parts would expand simultaneously as the balloon is expanding. In practical surgical applications, the prosthetic valve may fall off from such a simultaneous expansion of the overall balloon during operation, exposing the patient to serious safety hazards.

### SUMMARY OF THE INVENTION

It is an object of the present invention to provide a balloon catheter apparatus for dilating an aortic valve and a system. The expandable members can be independently controlled to expand as required by a particular surgical procedure, to not only dilate a prosthetic valve in a desirable way, but also facilitate locating of the prosthetic valve. In this way, the prosthetic valve can be prevented from falling off.

In order to overcome the problem of simultaneously overall expansion associated with conventional balloons, which may lead to falling off of a prosthetic valve during operation, there is provided herein a balloon catheter apparatus for dilating an aortic valve, the expandable assembly in the apparatus comprises three independently expandable members that are arranged in a predefined direction, each expandable member can be independently controlled to expand by a catheter assembly; during a particular surgical procedure, a corresponding expandable member can be independently controlled to expand as required by the surgical procedure so as to not only dilate a prosthetic valve, but also facilitate locating of the prosthetic valve. Thus, the prosthetic valve can be prevented from falling off.

The balloon catheter apparatus of the present invention comprises the catheter assembly and the expandable assembly.

The expandable assembly is disposed on the catheter assembly and comprises three expandable members that are independent and are arranged along an axial direction of the catheter assembly, wherein the three expandable members are a distal expandable member, a middle expandable member and a proximal expandable member. The middle expandable member is configured for placement of a prosthetic valve thereon.

The catheter assembly comprises a distal catheter, a middle catheter and a proximal catheter each being configured to transfer an inflation medium. The distal catheter communicates with an inflation cavity of the distal expandable member. The middle catheter communicates with the middle expandable member, and the proximal catheter communicates with the proximal expandable member.

Additionally, the expandable assembly may comprise at least three balloons which are arranged on the catheter assembly along the axial direction thereof, the at least three balloons are divided into three sets in the direction in which they are arranged, wherein the three sets of balloons serve as the distal expandable member, the middle expandable member and the proximal expandable member sequentially from a distal end to a proximal end of the catheter assembly in the axial direction thereof.

Additionally, the distal expandable member and the proximal expandable member may be configured such that, in an unexpanded configuration, a maximum radial dimension of the distal expandable member and the proximal expandable member is greater than an inner radial dimension of the prosthetic valve placed on the middle expandable member.

Additionally, the distal expandable member and the proximal expandable member may be configured such, in case of the prosthetic valve being placed on the middle expandable member, a portion of a proximal end of the distal expandable member and a portion of a distal end of the proximal expandable member are crimped within the prosthetic valve.

Additionally, any two of the distal catheter, the middle catheter and the proximal catheter may be located within the remaining catheter.

Additionally, the balloon catheter apparatus may further comprise a connector having a distal medium passage, a middle medium passage and a proximal medium passage, a distal end of the distal medium passage being in communication with a proximal end of the distal catheter, a distal end of the middle medium passage being in communication with a proximal end of the middle catheter, a distal end of the proximal medium passage being in communication with a proximal end of the proximal catheter.

Additionally, proximal ends of the distal medium passage, the middle medium passage and the proximal medium passage may diverge from one another to form a distal connecting port, a middle connecting port and a proximal connecting port, respectively, each of the distal connecting port, the middle connecting port and the proximal connecting port being connected to medium supply devices.

Additionally, the middle catheter and the proximal catheter may be located within the distal catheter.

Additionally, the catheter assembly may further comprise a guidewire catheter located within the distal catheter, wherein the connector further has a guidewire passage, a distal end of the guidewire passage is connected to a proximal end of the guidewire catheter.

The present invention also provides a catheter system for expanding a balloon, which comprises the balloon catheter apparatus as defined above and a prosthetic valve placed on the middle expandable member.

In summary, the present invention provides a balloon catheter apparatus for dilating an aortic valve, which includes a catheter assembly and an expandable assembly. The expandable assembly is disposed on the catheter assembly and includes three independent expandable members arranged along an axial direction of the catheter assembly, the three expandable members are a distal expandable member, a middle expandable member and a proximal expandable member. The middle expandable member is configured for placement of a prosthetic valve thereon. The catheter assembly includes a distal catheter, a middle catheter and a proximal catheter each being configured to transfer an inflation medium. The distal catheter communicates with an inflation cavity of the distal expandable member. The middle catheter communicates with the middle expandable member, and the proximal catheter communicates with the proximal expandable member.

With this arrangement, the three expandable members form distinct sections, the section of the middle expandable member is configure to place a prosthetic valve thereon. The distal and proximal expandable members are located at two ends of the placed prosthetic valve. These expandable members can be independently controlled to expand in a sequence as required by a particular surgical procedure. After the prosthetic valve is delivered to a target site and before it is deployed, the distal and proximal expandable members may be expanded first to limit the prosthetic valve both proximally and distally to prevent its unwanted axial displacement that may be caused by blood flow or other reasons. After that, the middle expandable member may be expanded to expand the prosthetic valve to a shape suitable for deployment. In this process, when the patient is found with special lesion anatomy, which requires independently adjusting radial dimensions of one or more of distal, middle and proximal sections of the prosthetic valve, degrees of expansion of corresponding ones of the expandable members may be independently controlled to address the demand. As these expandable members can be independently controlled to expand in a desired order to desired degrees, deployment of the prosthetic valve can be controlled more precisely in terms of location and shape. This allows a surgeon to modify and optimize the deployment of a prosthetic valve according to a patient's anatomical characteristics so that the prosthesis can be deployed in a shape more suitable for the patient's anatomy. Additionally, locating a prosthetic valve with the expandable members negates the conventional need of support structures located at the proximal and distal ends of a balloon, allowing the expandable members to have a simpler structure.

According to the present invention, balloons may be selected as units of the expandable assembly, and the distal, middle and proximal expandable members may be accordingly implemented as three balloons. Compared with the use of a single balloon, which is associated with the problem of overall expansion, using three independently expandable balloons provides for higher operability and controllability. The expansion of each balloon can be controlled simply by transfer of a commonly used liquid or gaseous inflation medium. After balloon expansion, it can facilitate the discharge of the inflation medium through its own contraction characteristics, aiding in the balloon's contraction.

Furthermore, dimensional parameters of the balloons may be configured so that in case of a prosthetic valve being placed on the middle expandable member, a maximum outer diameter of the distal expandable member is greater than a distal inner diameter of the prosthetic valve and a maximum outer diameter of the proximal expandable member is greater than a proximal inner diameter of the prosthetic valve. In this way, it can form a limit for the prosthetic valve in an unexpanded configuration, preventing the prosthetic valve from axial displacement during delivery within the sheath, thereby eliminating impact on a deployment shape of the prosthetic valve. In order to deploy the prosthetic valve, the distal and proximal expandable members may be expanded first, ensuring that the distal expandable member maintains a maximum radial dimension in a first direction greater than the distal inner diameter of the prosthetic valve and that the proximal expandable member maintains a maximum radial dimension in the first direction greater than the proximal inner diameter of the prosthetic valve.

### BRIEF DESCRIPTION OF THE DRAWINGS

Fig. 1 shows a schematic structural view of a conventional balloon catheter for dilating an aortic valve.
Fig. 2 shows a schematic structural view of a balloon in an expanded configuration in the conventional balloon catheter for dilating an aortic valve.
Fig. 3 shows a schematic structural view of a balloon catheter apparatus for dilating an aortic valve according to an embodiment of the present invention.
Fig. 4 shows a schematic structural view of an expandable assembly in an expanded configuration in a balloon catheter apparatus for dilating an aortic valve according to an embodiment of the present invention.
Fig. 5 shows a schematic cross-sectional view of a catheter assembly according to an embodiment of the present invention.
Fig. 6 shows a schematic cross-sectional view of a connector structure according to an embodiment of the present invention.

In these figures,
10, a catheter assembly; 11, a distal catheter; 12, a middle catheter; 13, a proximal catheter; 14, a guidewire catheter;
20, an expandable assembly; 21, a distal expandable member; 22, a middle expandable member; 23, a proximal expandable member;
30, a connector; 31, a distal medium passage; 32, a middle medium passage; 33, a proximal medium passage; 34, a guidewire passage; 311, a distal connecting port; 312, a middle connecting port; 313, a proximal connecting port;
40, a prosthetic valve;
51, a catheter; 52, a balloon; 521, a distal part; 522, a proximal part; 523, a waist part; and 53, a support.

### DETAILED DESCRIPTION

Balloon catheter apparatuses for dilating an aortic valve constructed in accordance with specific embodiments of the present invention will be described below in greater detail with reference to the accompanying drawings. From the following description, advantages and features of the application will become more apparent. Note that the figures are provided in a very simplified form not necessarily drawn to exact scale for the only purpose of helping in describing the embodiments in a convenient and clear way.

As used herein, the terms "proximal end" and "distal end" are employed to describe orientations, positions and directions of components of a product or actions thereon relative to one another, as viewed by a surgeon using the product. "Proximal end" is usually used to describe an end closer to the surgeon, in contrast to "distal end" being usually used to describe an end that enters the patient first, during normal operation of the product, although these terms are not intended to be limited to being used in such a way. An "axial direction" of a catheter assembly is defined as a direction at a certain location thereon along its axis from the distal end to proximal end. In other words, the axial direction is actually a direction of extension of the catheter assembly along its own length. The axial direction and the distal-to-proximal direction of the catheter assembly are not definite. Due to the bending of the catheter assembly during the delivery process along the artery, the axial direction at the location where the expansion component is installed on the catheter assembly, or the direction from the distal end to the proximal end of the catheter assembly, changes adaptively based on the bending shape. In addition, a radial direction of the catheter assembly is defined as a direction radiating from its center normally to its axial direction. When the catheter assembly is a round catheter, the radial direction is as per se. In case the catheter assembly is a non-round catheter, the radial direction is defined along the radius of a circumscribed or inscribed circle of its cross-section. An inner radial dimension of a prosthetic valve is defined as its inner diameter when the prosthesis is circular, or as the diameter of an inscribed circle thereof if the prosthesis is polygonal or otherwise shaped.

Further, the term "circumferential", when used to describe a balloon, generally refers to a direction about an axis thereof. As used herein, the singular forms "a", "an" and "the" include plural referents. The term "or" is generally employed in the sense of "and/or", "several" is generally employed in the sense of "at least one" and "at least two" is generally employed in the sense of "two or more". In addition, the terms "first", "second" and "third" are intended only for illustration and are not to be construed as denoting or implying relative importance, or as implicitly indicating the numerical number of the referenced items. Accordingly, defining an item with "first", "second" or "third" is an explicit or implicit indication of the presence of one or at least two such items. Further, as used herein, the terms "mounting", "coupling", "connecting", "disposing" and any variants thereof should be interpreted in a broad sense. Moreover, when an element is referred to as being "disposed" on another element, this is generally intended to only mean that there is a connection, coupling, engagement or transmission relationship between the two elements, which may be either direct or indirect with one or more intervening elements, and should not be interpreted as indicating or implying a particular spatial position relationship between them. That is, the element may be located inside, outside, above, under, beside, or at any other location relative to the other element, unless the context clearly dictates otherwise. Those of ordinary skill in the art can understand the specific meanings of the above-mentioned terms herein, depending on their context. Furthermore, the directional terms such as "above", "below", "upper", "lower", "upward", "downward", "left", "right", and the like are used in relation to the illustrative embodiments as they are depicted in the figures, the upward or upper direction being toward the top of the corresponding figure and the downward or lower direction being toward the bottom of the corresponding figure.

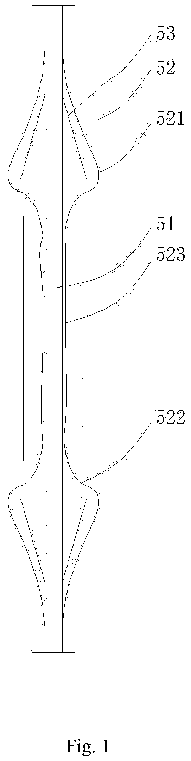

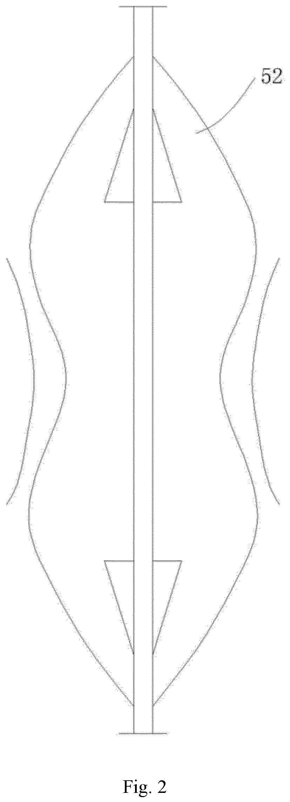

Referring to Figs. 1 and 2, a conventional balloon catheter for dilating an aortic valve includes a catheter 51 and a balloon 52 provided on the catheter. The catheter 51 has a medium transfer port, which communicates with an inner cavity of the balloon to allow a gas or liquid to be introduced to inflate the balloon. The balloon 52 is divided lengthwise into a distal part 521, a proximal part 522 and a waist part 523. The waist part is usually straight or recessed, and supports 53 are provided internally at the distal and proximal ends of the balloon 52. A prosthetic valve can be crimped on the waist part of the balloon between the two supports 53, and the supports are used to restrict the crimped prosthetic valve from unwanted axial displacement when it is being advanced for delivery, which may make it unsuccessful to dilate the valve by expanding the balloon because of the displacement of the valve. As the balloon 52 is an integral structure, the distal, proximal and waist parts would all expand simultaneously as the balloon is expanding. However, in practical surgical applications, as a consequence of such overall expansion, the prosthetic valve may displace or even fall off when its inner diameter is increased beyond a radial dimension of the supports 53. This would expose the patient to serious safety hazards. The integral balloon cannot independently control the expansion of the distal, proximal and waist parts, the valve has to be deployed in an overall manner without any portion thereof being independently controllable in this process and therefore suffers from a lack of adaptiveness to the patient's anatomy.

As can be seen from the above, which portions of a balloon expand and an order of expansion of these portions are affected by how the balloon is structured and, in turn, greatly affect the reliability of the procedure that uses the balloon and its adaptiveness to the patient. Therefore, in embodiments of the present invention, there is provided an apparatus capable of controlling whether an individual portion of a balloon expands or not, as well as an order of expansion of different portions thereof.

These embodiments overcome the problem of overall expansion with the conventional balloon, which may lead to falling off of a prosthetic valve. Accordingly, a balloon catheter apparatus for dilating an aortic valve is provided, which includes an expandable assembly with three independent expandable members arranged in a predefined direction. A catheter assembly is able to independently control expansion of the individual expandable members. In a practical surgical procedure, one or more of the expandable members can be expanded, as required by the procedure, to not only dilate a prosthetic valve in a desirable way, but also facilitate locating of the prosthetic valve in the predefined direction. As such, the prosthetic valve can be prevented from falling off.

A balloon catheter apparatus for dilating an aortic valve according to an embodiment of the present invention includes a catheter assembly 10 and an expandable assembly 20.

The expandable assembly 20 is disposed on the catheter assembly 10 and has three independent expandable members arranged from a distal end to a proximal end of the catheter assembly 10, which are a distal expandable member 21, a middle expandable member 22 and a proximal expandable member 23. Each expandable member comprises an inflation cavity, and the middle expandable member is configured for placement of a prosthetic valve 40 thereon. For example, the prosthetic valve may be crimped on the middle expandable member. How the prosthetic valve is placed may depend on how it is structured.

The catheter assembly 10 includes distal, middle and proximal transfer ports for supply of an inflation medium. The distal transfer port communicates with the inflation cavity of the distal expandable member 21 so that an inflation medium can be independently supplied to inflate the distal expandable member 21. The middle transfer port communicates with the inflation cavity of the middle expandable member so that an inflation medium can be independently supplied to inflate the middle expandable member. The proximal transfer port communicates with the inflation cavity of the proximal expandable member 23 so that an inflation medium can be independently supplied to inflate the proximal expandable member 23.

In one embodiment, the expandable assembly 20 may be a balloon structure made of an elastic material with a certain degree of stretchability. The material of the balloon may be selected from polyesters, polyurethanes, thermoplastic elastomers, polyethylene or polyolefin copolymers, other polymer materials, and combinations thereof. As a suitable inflation medium for use with the balloon, physiological saline or a gas that is harmless to humans may be used. The gaseous or liquid inflation medium can be injected into the balloon to cause its expansion. Such expansion is physical and occurs by virtue of the stretchability of the balloon material, and the inflation medium itself does not affect the material's stability at all.

In an alternative embodiment, the expandable assembly 20 may be expanded chemically. For example, a first chemical may be pre-filled in an inflation cavity of the expandable assembly 20, and a second chemical may be introduced into the same inflation cavity through the catheter assembly 10 so that the two chemicals meet and react with each other to give rise to a gas or gaseous substance, which fills and expands the expandable assembly 20.

In another alternative embodiment, the expandable assembly 20 may be expanded thermally. However, based on the existing and relatively mature application technology, the expansion is usually achieved by using a balloon in combination with a gaseous or liquid expansion medium. Under the premise of meeting the requirements for prosthetic valve expansion, this can be achieved with a simple medium and a simple expansion structure, which helps to simplify the overall device structure. Moreover, using a gaseous or liquid expansion medium is relatively safe for the human body. For example, if physiological saline is used as the medium, even in the event of a leak, it would not cause any substantial harm to the patient

The expandable members may be separate members. For example, each of them may consist of a separate balloon, or of multiple expandable units. For example, each of the expandable members may be made of multiple separate balloons. In this case, each balloon in the expandable member may communicate with a corresponding transfer port.

The catheter assembly 10 may be implemented as a single catheter. In this case, the distal, middle and proximal transfer ports may be all provided on the single catheter. When it is required to supply the three transfer ports with separate inflation medium, such control of the three transfer ports that they are independently opened and closed is necessary. In this way, the expandable members may be controlled independently so that one or more of the transfer ports can be opened to cause expansion of the respective expandable member(s).

In an alternative embodiment, the catheter assembly 10 may be composed of multiple catheters, for example, three catheters. In this case, each of the transfer ports may be provided on a respective one of the catheters, which is in communication with a respective one of the distal expandable member 21, the middle expandable member 22 and the proximal expandable member 23 and connected to a respective external inflation medium supply device. With this arrangement, the catheters can be supplied with separate inflation medium, and the expandable members can be controlled independently.

In another alternative embodiment, the catheter assembly 10 may be implemented as a single composite catheter defining three transfer lumens, and the distal, middle and proximal transfer ports may be provided on the catheter so as to communicate with the respective transfer lumens. Moreover, the three transfer lumens may be connected to respective external inflation medium supply devices. This embodiment operates in a similar way to, but provides higher degree of integration than, the catheter assembly 10 consisting of multiple catheters.

As the catheter assembly 10 is to be advanced through an artery, it is provided as a non-rigid structure desirably with good ability to be advanced through bends and bent and flexed at will. Optionally, the catheter assembly 10 is made of a polymer material. For example, it may be fabricated from a thermoplastic polyurethane (TPU) elastomeric rubber reinforced with metal filaments, a polyether block amide resin (Pebax) or a nylon material with good plasticity. Alternatively, it may be a braided metal tube. Each of the distal, middle and proximal transfer ports in the catheter assembly 10 is connected to an external syringe or other form of medium supply device which is to supply a liquid or gaseous medium. In case of the expandable members being implemented as balloon structures, after the prosthetic valve is deployed, an internal pressure of the catheter assembly 10 may be released so that the expandable members contract by their own elastic stretchability to squeeze out the medium or media therein. When the expandable members themselves are not elastically stretchable as such, the medium supply devices connected to the respective transfer ports are desired to be capable of evacuating air from the catheter assembly 10 to create a negative pressure environment therein, which forces the inflation medium or media out of the expandable members through the transfer ports and the catheter assembly 10. As a result, the expandable members collapse, facilitating their withdrawal along with the catheter assembly 10.

The aforementioned balloon catheter apparatus for dilating the aortic valve is designed with three expandable members, forming distinct sections. The middle expandable member is used for positioning the prosthetic valve 40 thereon, while the distal expandable member 21 and the proximal expandable member 23 are located at two ends of the placed prosthetic valve 40. Each expandable member can be independently controlled for its expansion, allowing the sequence of expansion of the expandable members to be selected based on the surgical needs during the surgical procedure. After the prosthetic valve is positioned but before it is deployed, the distal and proximal expandable members 21, 23 can be inflated first to secure the proximal and distal ends of the prosthetic valve, preventing it from moving due to blood flow or other factors. Then, the middle expandable member 22 can be inflated to expand the prosthetic valve into its proper shape. In this process, when the patient is found with special lesion anatomy, which requires separately adjusting radial dimensions of one or more of distal, middle and proximal sections of the prosthetic valve, degrees of expansion of corresponding ones of the expandable members may be separately controlled to address the demand. As these expandable members can be separately controlled to expand in a desired temporal order to desired degrees, deployment of the prosthetic valve can be controlled more precisely in terms of location and shape. This allows a surgeon to modify and optimize the deployment of a prosthetic valve according to a patient's anatomical characteristics so that the prosthesis can be deployed in a shape more suitable for the patient's anatomy. Additionally, locating a prosthetic valve with the expandable members negates the conventional need of supports internally located at proximal and distal ends of a balloon, allowing the expandable members to have a simpler structure.

Further, the expandable assembly 20 may include at least three balloons arranged on the catheter assembly 10 along an axial direction thereof. These balloons are divided into three sets along the direction in which they are arranged. Sequentially from the distal end to the proximal end of the catheter assembly along its axial direction, the three sets of balloons respectively serve as the distal expandable member 21, the middle expandable member 22 and the proximal expandable member 23.

The balloons may be adhesively bonded to the catheter assembly 10. The balloon is chosen as the unit of the expandable assembly 20 because, first, balloon technology is relatively mature, and second, the expansion and contraction of the balloon are easy to control. The balloons can be inflated with a commonly used liquid or gaseous inflation medium. Moreover, after being expanded, a balloon can easily contract by its own stretchability to discharge an inflation medium. The more balloons are used, the more precisely a deployment shape of the prosthetic valve 40 can be controlled. The number of balloons in each expandable member may be determined as required by practical surgical procedures.

Further, the distal 21 and 23 proximal expandable members may be configured with a maximum radial dimension in an unexpanded configuration, which is greater than an inner radial dimension of the prosthetic valve on the middle expandable member 22. This enables the distal 21 and 23 proximal expandable members to restrict the prosthetic valve in the axial direction of the catheter assembly 10 from its distal to proximal end.

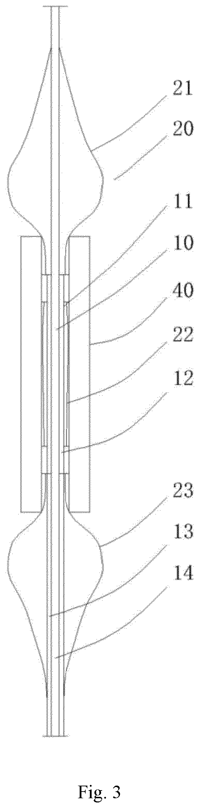

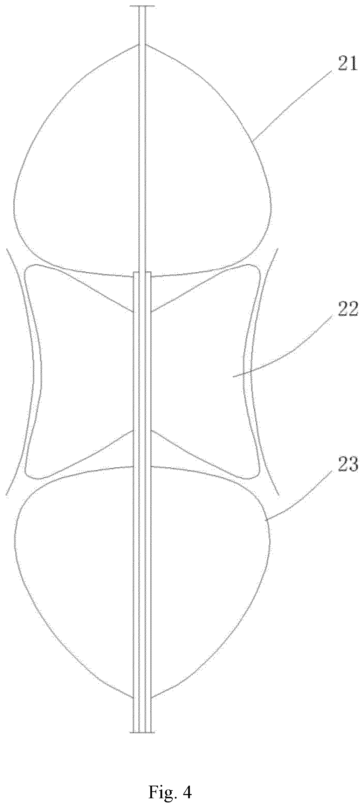

Referring to Figs. 3 and 4, both the distal 21 and 23 proximal expandable members may be balloon structures, and a prosthetic valve may be crimped on the middle expandable member 22 such that, a maximum outer diameter of the distal expandable member 21 is greater than an inner radial dimension of the prosthetic valve at its distal end and that a maximum outer diameter of the proximal expandable member 23 is greater than an inner radial dimension of the prosthetic valve at its proximal end. In this way, when the prosthetic valve is delivered in an unexpanded configuration within a sheath, it can be restricted from unwanted axial displacement, which may affect a final deployment shape of the prosthetic valve. In order to deploy the prosthetic valve, the distal 21 and proximal 23 expandable members may be expanded first, ensuring that the distal expandable member 21 maintains a maximum radial dimension in a first direction greater than the inner radial dimension of the prosthetic valve at the distal end, and that the proximal expandable member 23 maintains a maximum radial dimension in the first direction greater than the inner radial dimension of the prosthetic valve at the proximal end.

Further, the distal 21 and 23 proximal expandable members may be configured such that, in case of a prosthetic valve being placed on the middle expandable member 22, a portion of a proximal end of the distal expandable member 21 and a portion of a distal end of the proximal expandable member 23 are both crimped within the prosthetic valve.

As shown in Fig. 3, a proximal portion of the distal expandable member 21 may be collapsed and received in a distal section of the prosthetic valve, and a distal portion of the proximal expandable member 23 may be collapsed and received in a proximal section of the prosthetic valve. This can ensure good overall stability of the apparatus.

Further, the catheter assembly 10 may include a distal catheter 11, a middle catheter 12 and a proximal catheter 13. The distal transfer port may be provided on the distal catheter 11, the middle transfer port on the middle catheter 12, and the proximal transfer port on the proximal catheter 13.

The catheter assembly 10 may be made up of three separate catheters, for example, the distal catheter 11, the middle catheter 12 and the proximal catheter 13, each of which is connected to an external syringe or other form of medium supply device capable of injecting or discharging a liquid or gaseous medium to independently control a respective one of the expandable members. In this way, it is unnecessary to separately control opening and closing of the individual transfer ports, simplifying the control structure.

Further, the expandable assembly 20 may include three balloons disposed on the catheter assembly 10 in the direction from the distal end to the proximal end thereof. In this case, the three balloons may respectively serve as the distal expandable member 21, the middle expandable member 22 and the proximal expandable member 23, in the distal-to-proximal direction in which they are arranged.

For a balloon, its size, outer diameter and pressure withstanding capacity may be used as basic metrics for assessing its performance. Referring to Fig. 3, an outer diameter of the balloon corresponding to the distal expandable member 21 measured at the middle thereof may be greater than a distal inner diameter of a prosthetic valve. Moreover, an outer diameter of the balloon corresponding to the proximal expandable member 23 measured at the middle thereof may be greater than a proximal inner diameter of a prosthetic valve. Further, the balloon corresponding to the middle expandable member may have an outer diameter less than the inner diameters of the prosthetic valve. Of course, the middle expandable member may instead have an outer diameter greater than the inner diameters of the prosthetic valve, and the prosthetic valve may be crimped around the middle expandable member through external force. In addition, the compliance of a balloon is closely related to the material from which it is made. Common examples of the material include nylon and special polyethylene. Balloons made of special polyethylene possess good physical properties including a very small thickness, low compliance and high pressure resistance. Although low compliance can prevent a balloon from expanding excessively at both ends to possibly cause tears in a blood vessel wall, it means inferior plasticity. Therefore, semi-compliant nylon balloons are preferred.

Further, two of the distal catheter 11, the middle catheter 12 and the proximal catheter 13 may be received in the remaining catheter.

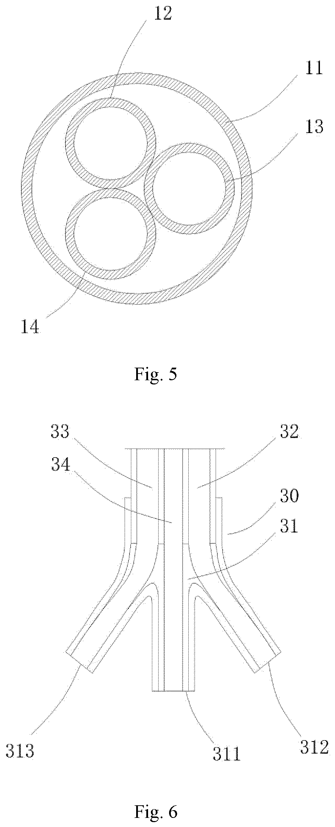

With additional reference to Fig. 5, two of the catheters may be received in the remaining one so that only the outermost catheter is visible. This can ensure that the catheter assembly can be provided as a smooth structure with a specifically-shaped outer contour. Optionally, the outermost catheter is a round tube with a smooth, circular outer surface. For the other two catheters located internally, since they will not be brought into direct contact with the wall of a human artery, the present invention is not limited to any particular shape or outer contour of either of them. This allows the catheter assembly to have a high degree of integration, which can facilitate advancement within an artery.

The balloon catheter apparatus may further include a connector 30 with a distal medium passage 31, a middle medium passage 32 and a proximal medium passage 33. A distal end of the distal medium passage 31 may communicate with a proximal end of the distal catheter 11. A distal end of the middle medium passage 32 may communicate with a proximal end of the middle catheter 12. A distal end of the proximal medium passage 33 may communicate with a proximal end of the proximal catheter 13.

Referring to Fig. 6, the distal medium passage 31, the middle medium passage 32 and the proximal medium passage 33 may be integrated in the connector 30. The proximal end of the catheter assembly 10 may be directly connected to the connector so that the medium passages are brought into communication with the respective catheters. Connecting the catheters in the catheter assembly 10 using this integrated connector provides centralized connection which allows for easier management.

Further, proximal ends of the distal medium passage 31, the middle medium passage 32 and the proximal medium passage 33 may diverge away from one another to form a distal connecting port 311, a middle connecting port 312 and a proximal connecting port 313, respectively, where they are respectively connected to the medium supply device.

Referring to Fig. 6, the distal medium passage 31, the middle medium passage 32 and the proximal medium passage 33 may be all round channels, and the connector 30 has three branch structures with each forming an interface. These interfaces may be threaded or other known types of interfaces. The arrangement of the specialized interfaces makes it easy to connect to a medium supply device.

Further, the middle catheter 12 and the proximal catheter 13 may be received in the distal catheter 11.

Referring to Fig. 3, the distal catheter 11 may be connected to the distal expandable member 21. To this end, the distal catheter 11 may extend more distally so that the middle and proximal catheters are both entirely received in the distal catheter 11. Additionally, the middle and proximal transfer ports may be both provided in a wall of the distal catheter 11 so as to come into communication with the middle and proximal catheters, respectively. In this way, the distal expandable member 21, the middle expandable member 22 and the proximal expandable member 23 can be all disposed on the distal catheter 11, and the middle expandable member 22 communicates with the middle transfer port, the proximal expandable member 23 communicates with the proximal transfer port, and the distal transfer port on the distal catheter 11 may be brought into communication with the distal expandable member 21.

The catheter assembly 10 may further include a guidewire catheter 14 disposed within the distal catheter 11, and the connector structure 30 may further have a guidewire passage 34. A distal end of the guidewire passage may communicate with a proximal end of the guidewire catheter 11.

Referring to Figs. 5 and 6, according to embodiments of the present invention, the distal catheter 11, the middle catheter 12, the proximal catheter 13 and the guidewire catheter 14 are preferably all round tubes. The middle catheter 12, the proximal catheter 13 and the guidewire catheter 14 are triangularly arranged within the distal catheter 11, and are externally tangent to one another. Additionally, each of the middle catheter 12, the proximal catheter 13 and the guidewire catheter 14 may be internally tangent to the distal catheter 11. This can ensure that the four catheters are stably located relative to one another both radially and circumferentially. In other embodiments, the middle catheter 12, the proximal catheter 13 and the guidewire catheter 14 may be arranged in different manners relative to the distal catheter 11, as required by practical applications. The guidewire passage 34 may extend within the distal medium passage 31, and an annular opening between the guidewire passage 34 and the distal medium passage 31 may provide the distal connecting port 311. The guidewire passage is configured for the insertion therethrough of a guidewire for guiding the catheter assembly 10.

In embodiments of the present invention, there is also provided a catheter system for expanding a balloon, which includes the balloon catheter apparatus as defined above for dilating the aortic valve and a prosthetic valve 40 placed on the middle expandable member 22. The prosthetic valve 40 generally includes a stent, a membrane defining a flow path and other components. The prosthetic valve is usually wrapped around the middle expandable member. However, it may also be placed in a different manner, depending on its structure.

The embodiments disclosed herein are described in a progressive manner, with the description of each embodiment focusing on its differences from others. Cross-reference can be made between the embodiments for their common or similar features.

The description presented above is merely that of some preferred embodiments of the present invention and does not limit the scope thereof in any sense. Any and all changes and modifications made by those of ordinary skill in the art based on the above teachings fall within the scope as defined in the appended claims.

## Claims

1. A balloon catheter apparatus for dilating an aortic valve, comprising a catheter assembly and an expandable assembly,
wherein the expandable assembly is provided on the catheter assembly, wherein the expandable assembly comprises three expandable members that are independent and are arranged along an axial direction of the catheter assembly, wherein the three expandable members are a distal expandable member, a middle expandable member and a proximal expandable member, respectively, and wherein the middle expandable member is configured for placement of a prosthetic valve thereon,
wherein the catheter assembly comprises a distal catheter, a middle catheter and a proximal catheter each being configured to transfer an inflation medium, and wherein: the distal catheter is in communication with an inflation cavity of the distal expandable member; the middle catheter is in communication with the middle expandable member; and the proximal catheter is in communication with the proximal expandable member.

2. The balloon catheter apparatus of claim 1, wherein the expandable assembly comprises at least three balloons which are arranged on the catheter assembly along the axial direction thereof, wherein the at least three balloons are divided into three sets in a direction in which the balloons are arranged, wherein the three sets of balloons serve as the distal expandable member, the middle expandable member and the proximal expandable member sequentially from a distal end to a proximal end of the catheter assembly in the axial direction thereof.

3. The balloon catheter apparatus of claim 1, wherein the distal expandable member and the proximal expandable member are configured such that, in an unexpanded configuration, a maximum radial dimension of the distal expandable member and the proximal expandable member is greater than an inner radial dimension of the prosthetic valve placed on the middle expandable member.

4. The balloon catheter apparatus of claim 1, wherein the distal expandable member and the proximal expandable member are configured that, in case of the prosthetic valve being placed on the middle expandable member, a portion of a proximal end of the distal expandable member and a portion of a distal end of the proximal expandable member are crimped within the prosthetic valve.

5. The balloon catheter apparatus of claim 1, wherein any two of the distal catheter, the middle catheter and the proximal catheter are located within a remaining catheter.

6. The balloon catheter apparatus of claim 1, further comprising a connector, wherein the connector comprises a distal medium passage, a middle medium passage and a proximal medium passage, and wherein: a distal end of the distal medium passage is in communication with a proximal end of the distal catheter; a distal end of the middle medium passage is in communication with a proximal end of the middle catheter; and a distal end of the proximal medium passage is in communication with a proximal end of the proximal catheter.

7. The balloon catheter apparatus of claim 6, wherein proximal ends of the distal medium passage, the middle medium passage and the proximal medium passage diverge away from one another to form a distal connecting port, a middle connecting port and a proximal connecting port, respectively, and wherein each of the distal connecting port, the middle connecting port and the proximal connecting port is connected to a medium supply device.

8. The balloon catheter apparatus of claim 7, wherein the middle catheter and the proximal catheter are located within the distal catheter.

9. The balloon catheter apparatus of claim 8, wherein the catheter assembly further comprises a guidewire catheter located within the distal catheter, wherein the connector further comprises a guidewire passage, and wherein a distal end of the guidewire passage is connected to a proximal end of the guidewire catheter.

10. A catheter system for expanding a balloon, comprising the balloon catheter apparatus of any one of claims 1 to 9 and a prosthetic valve placed on the middle expandable member.
